# EUROPEAN PATENT APPLICATION

(11) **EP 3 037 816 A1**
(43) Date of publication of application: **29.06.2016**
(21) Application number: 14004388.6
(22) Date of filing: 23.12.2014
(51) Int. Cl.: G01N 33/50, A61K 35/17, C12N 5/0783

(54) **Method for the identification of transiently Foxp3 negative regulatory T-cells from human peripheral blood**

(71) Applicant: Julius-Maximilians-Universität Würzburg, 97070 Würzburg (DE)
(72) Inventor: Hünig, Thomas, 97286 Winterhausen (DE)
(74) Representative: Jungblut, Bernhard Jakob

(57) **Abstract**

The invention relates to a method for determination of the frequency of regulatory T-cells in samples obtained from human blood and to methods for the preparation of compositions comprising predetermined amount of regulatory T-cells. The invention is based on the conception that a large fraction of regulatory T-cells present in human peripheral blood do not express detectable amounts of Foxp3, the master transcription factor used for identification of regulatory T-cells, as a result of cytokine deprivation outside of the tissue context.

## Description

### FIELD OF THE INVENTION

The invention relates to a method for the improved determination of regulatory T-cells (Treg cells) in human blood samples, wherein peripheral blood mononuclear cells (PBMC) from the sample are cultivated and wherein Treg cells are determined by detection of Forkhead-Box-Protein P3 (Foxp3) and CD25 expression.

### BACKGROUND OF THE INVENTION AND STATE OF THE ART

Regulatory T-cells (Treg cells) play a key role in the control of autoimmunity, inflammation and other immunopathologies. Their differentiation and function depends on the expression of the master transcription factor Forkhead-Box-Protein P3 (Foxp3). This is illustrated by the multiple autoimmune diseases experienced by patients with the IPEX syndrome, a genetic defect in the gene encoding Foxp3 (van der Vliet, H.J. and Nieuwenhuis, E. E., IPEX as a result of mutations in FOXP3. Clin Dev Immunol 2007. 2007: 89017.). Accordingly, comparing the frequency of Treg cells in peripheral blood of patients with immune system-related disorders to those of healthy volunteers, and following the dynamics of Treg frequencies under treatment with experimental or established immunomodulatory drugs has become a key analytical and diagnostic parameter; for examples see Dalla Libera, D. et al., T regulatory cells are markers of disease activity in multiple sclerosis patients. PLoS One 2011. 6: e21386, Jamshidian, A. et al., Biased Treg/Th17 balance away from regulatory toward inflammatory phenotype in relapsed multiple sclerosis and its correlation with severity of symptoms, J. Neuroimmunol 2013.262: 106-112, and Kawashiri, S. Y., et al., CD4+CD25(high)CD127(low/-) Treg cell frequency from peripheral blood correlates with disease activity in patients with rheumatoid arthritis. J Rheumatol 2011.38: 2517-2521.

Furthermore, Treg cells are isolated from peripheral blond in experimental therapies, expanded in cell culture and re-infused into patients for therapeutic purposes (Edinger, M. and Hoffmann, P., Regulatory T cells in stem cell transplantation: strategies and first clinical experiences. Curr Opin Immunol 2011. 23: 679-684).

Regulatory T-cells are a subset of CD4 expressing T-cells (Sakaguchi, S. et al., Naturally arising Foxp3-expressing CD25+CD4+ regulatory T cells in self-tolerance and autoimmune disease, Curr Top Microbiol Immunol 2006. 305: 57-66). They are phenotypically characterized by constitutive expression of CD25, the alpha chain of the interleukin-2 (IL-2) receptor, which on other T-cells is expressed only in response to stimulation, and the intracellular expression of Foxp3. While additional markers such as low expression of CD127 (Seddiki, N. et al., Expression of interleukin (IL)-2 and IL-7 receptors discriminates between human regulatory and activated T cells. J Exp Med 2006.203: 1693-1700) and CD45RA expression (Sakaguchi, S. et al.,FOXP3+ regulatory T cells in the human immune system. Nat Rev Immunol 2010.10: 490-500) have been used, CD 25 and Foxp3 remain indispensable for Treg definition. Accordingly, identification of regulatory T-cells relies on the flowcytometric detection of CD4+ CD25+ Foxp3+ cells among peripheral blood mononuclear cells (PBMC), using appropriate fluorochrome-labeled monoclonal antibodies for cell surface staining of CD4 and CD25, and, after fixation and permeabilization of the cells, for intracellular detection of Foxp3.

The contribution of Treg cells thus defined to the CD4 T-cell compartment in lymphoid tissues is about 5 -10%. In contrast, this frequency is much lower among peripheral blond CD4 T-cells (Sakaguchi, S. et al., D. A., FOXP3+ regulatory T cells in the human immune system. Nat Rev Immunol 2010.10: 490-500). The generally accepted explanation for differences in lymphocyte subset representation in lymphoid organs as compared to blood is a differential tendency of these subsets to enter the blond stream and recirculate before they re-enter a tissue compartment.

For most purposes, especially in diagnostic routine and in immunologic research, peripheral blood remains the only available source of immune cells. Accordingly, a flawed analysis of Treg cells from this compartment has far-reaching consequences in clinical practice as well as in preclinical research and clinical drug development.

In the document Zorn, E., et al., IL-2 regulates FOXP3 expression in human CD4+CD25+ regulatory T cells through a STAT-dependent mechanism and induces the expansion of these cells in vivo, Blood 2006.108:1571-1579, experiments are described to investigate whether Treg activity in humans could be improved by IL-2. In this study, PBMC cultures were incubated with IL-2 and an increase in Foxp3 protein was demonstrated by Western blot Analysis. This method, however, does not detect protein expression at the level of individual cells, but in a protein extract derived from a cell preparation containing a large number of cells, and therefore does not provide information about the frequency of Foxp3 expressing cells.

### TECHNICAL PROBLEM OF THE INVENTION

The technical problem underlying the instant invention is, thus, to provide means for the improved, in particular more precise, determination of Treg cells in samples obtained from human blood and to provide compositions containing Treg in a predetermined amount, wherein the accuracy of said amount is improved.

### PRINCIPLES OF THE INVENTION AND PREFERRED EMBODIMENTS

For solving these technical problems a first aspect of the invention is directed to a method for the in-vitro determination of regulatory T-cells (Treg cells) in a sample obtained from human blood with the following steps: A1) said sample is supplemented with a compound capable of activating the Signal Transducer and Activator of Transcription 5 (STAT5) and in an amount capable for such activation, and cultured for a predetermined cultivation duration under conditions which keep T-cells comprised in said sample viable, wherein the cultivation is carried out either during said supplementing or after said supplementing, A2) after cultivation of said sample an analytical compound specific for CD25 and/or Forkhead-Box-Protein P3 (Foxp3) is added to said sample and the cell frequency of CD25 and/or Foxp3 positive cells is determined in said sample. Frequency determination is in particular performed by determining the ratio of the amount of CD25 and/or Foxp3 positive cells to the amount of CD4 positive cells.

A second aspect of the invention resulting from the first aspect is directed to a method for preparing a composition, in particular a pharmaceutical composition, comprising a predetermined amount of viable Treg cells with the following steps: B1) a sample is obtained from human blood and subjected to an in-vitro cultivation under conditions which keep T-cells comprised in said sample viable, said cultivation being performed during or after supplementing said sample with a compound capable of activating the Signal Transducer and Activator of Transcription 5 (STAT5), B2) then an analytic compound specific for CD25 is added and the frequency of CD25 positive cells is determined, B3) then a fraction of said sample is taken, wherein the fraction is calculated from the frequency of CD25 positive cells such that the fraction comprises the predetermined amount of viable Treg cells, and said fraction is optionally prepared for administration, e.g. by fluorescence or magnetic bead mediated activation. Frequency determination is in particular performed by determining the ratio of the amount of CD25 positive cells to the amount of CD4 positive cells.

A third aspect of the invention resulting from the first aspect is directed to a method for preparing a composition, in particular a pharmaceutical composition, comprising a predetermined amount of viable Treg cells with the following steps: C1) a sample is obtained from human blood and subjected to an in-vitro cultivation under conditions which keep T-cells comprised in said sample viable, said cultivation being performed during or after supplementing said sample with a compound capable of activating the Signal Transducer and Activator of Transcription 5 (STAT5), C2) then an analytic compound specific for Foxp3 is added to a first fraction of said sample and the frequency of Foxp3 positive cells is determined, C3) then a second fraction of said sample is taken, wherein the fraction is calculated from the frequency of Foxp3 positive cells in the first fraction such that the second fraction comprises the predetermined amount of viable Treg cells, and said second fraction is optionally prepared for administration. Frequency determination is in particular performed by determining the ratio of the amount of Foxp3 positive cells to the amount of CD4 positive cells.

Herein the term frequency provides for the fraction of detected Treg cells among all CD4 positive cells in the sample and may be calculated as the fraction of the amount of CD 25 and/or Foxp3 positive cells to the amount of CD4 positive cells. Insofar it is desirable to detect the number and/or concentration of CD4 positive cells in a sample in case that absolute frequency values are desired. If, however, two or more samples of the same origin are tested and used, it is sufficient to detect relative frequencies, i.e. a measurement of CD4 positive cells is then not necessary in all cases.

The conception of the invention and preferred embodiments are described in the following.

Within the conception of the instant invention, an alternative explanation for the low frequency of Treg cells in peripheral blond was conceived based on the dependence of Foxp3 expression on the binding of certain cytokines, in particular the cytokine interleukin-2 (IL-2), to cell surface receptors on the Treg cells. Upon ligand binding, these receptors activate the Signal Transducer and Activator of Transcription 5 (STAT5), which dimerizes and translocates to the nucleus where it is required for the transcription of the Foxp3 locus (Passerini, L et al.., STAT5-signaling cytokines regulate the expression of FOXP3 in CD4+CD2S+ regulatory T cells and CD4+CD25-effector T cells. Int Immunol 2008. 20: 421-431). Together with other transcription factors, STAT5 then allows Foxp3 transcription and, in consequence, the formation of the Foxp3 protein (Zorn, E., et al., IL-2 regulates FOXP3 expression in human CD4+CD25+ regulatory T cells through a STAT-dependent mechanism and induces the expansion of these cells in vivo. Blood 2006.108:1571-1579).

Since the Foxp3 Protein has a short half life (van Loosdregt, J. et al., Regulation of Treg functionality by acetylation-mediated Foxp3 protein stabilization. Blood 2010.115: 965-974; Morawski, P. A., et al., Foxp3 protein stability is regulated by cyclin-dependent kinase 2, J Biol Chem 2013, 288. 24494-24502), it was speculated that the lack of availability of STAT5-activating cytokines, which are normally provided by neighboring cells in lymphoid and other tissues, but are undetectable in the blond of healthy individuals, leads to a transient down regulation of Foxp3 in Treg cells and escape from Foxp3 based flow-cytometric analysis. In mice, in vivo Blockade of IL-2 with neutralizing antibodies does indeed lead to a loss of Foxp3 expression in a fraction of, and to a reduction in Foxp3 expression levels in the remaining Treg cells (Rubtsov, Y. P., et al., Stability of the regulatory T cell lineage in vivo. Science 2010.329: 1667-1671). Accordingly, the hence unappreciated possibility was envisaged that human blood would contain Foxp3 expressing Treg cells, which had only recently entered the blood stream, and Foxp3 low and negative ones, which have down regulated Foxp3 expression after some time of cytokine withdrawal as recirculating cells. Since nothing is known about the time Treg cells spend in human blood during recirculation, it was completely open whether this speculation could be verified.

An important aspect within the instant inventions conception is the fact that the ability to transcribe the FOXP3 gene locus depends on the epigenetic imprinting of certain regulatory sequences, in particular the TSDR region, by de-methylation of cytokine nucleotides (Delacher, M., et al., Transcriptional Control of Regulatory T cells, Curr Top Microbiol Immunol 2014. 381: 83-124.). Thus, if STAT5, which is activated by signaling of the IL-2 receptor upon IL-2 binding, is again provided, Foxp3-negative Treg cells are enabled to produce the Foxp3 protein again, making them amenable to detection, whereas other T-cells remain Foxp3 negative.

Indeed, it was shown within the instant invention that when PBMC from healthy donors are either immediately analyzed for the presence of Treg cells using the marker combination CD4, CD25 and Foxp3, or are first incubated for various lengths of time in the presence of IL-2, the cytokine which plays a key role in the maintenance of Treg numbers in the organism, a surprisingly dramatic increase (up to 3 fold) in the frequency of Foxp3+ CD25+ CD4 T-cells is observed. This effect becomes visible as early as 6 hours after onset of stimulation with IL-2, and reaches a plateau after about 20 hours, is detectable with as little as 6 U/ml of IL-2 and is saturated at about 600 U/ml of IL-2. Importantly, not only the frequency of Foxp3 expressing cells is increased by this treatment, but also the expression intensity of Foxp3 as quantified by mean fluorescence intensity (MFI) determined by flow cytometric measurement with Foxp3-specific mAb. Together, these two effects greatly facilitate the identification of Treg cells for quantification and further characterization.

In addition to an up-regulation of Foxp3 in previously Foxp3 negative CD4 T-cells by overnight incubation with IL-2, there is a pronounced up-regulation of the level of CD25 expression on the same cells as indicated by an increase in both, frequency of CD25 expressing cells and expression level (MFI). This is explained by the fact that Foxp3 and STAT5 positively influence transcription of the CD25 Gen.

Since the CD25-high cells thus obtained are also Foxp3-high, IL-2 pre-treated PBMC are not only readily identified for further characterization by flow cytometry, they also are a superior source for the isolation of Treg cells by flow cytometry to be further used for experimental or therapeutic purposes based on CD25 expression in the CD4 T-cell subset. This is because Foxp3 itself cannot be used for viable cell sorting due to its intracellular expression which requires fixation and permeabilization for its detection by mAb.

The same effect is observed if instead of whole PBMC, which consist of various types of lymphocytes and monocytes, purified CD4 T-cells, of which Treg cells are a subset, are used, indicating a direct action of IL-2 on a subpopulation of the CD4 cells, i.e. the "cryptic" Treg cells.

Besides IL-2, the main driver of Treg homeostasis and Foxp3 expression, other cytokines which activate the STATS pathway such as 1L-7 and IL-15 can also be used to restore Foxp3 and CD25 expression in the Treg subset of CD4 T-cells.

An increase in CD4+ CD25+ Foxp3+ cells as a result of overnight culture in the presence of IL-2 could also be explained by proliferation of cells with this phenotype. This is, however, not the case as is illustrated by the proliferative history of the recovered cells as determined by flow cytometric analysis of PBMC labeled with a covalent fluorescent dye which is reduced to half its original intensity with each cell division.

Besides the cytokine-mediated up-regulation of Foxp3 in pre-existing Treg cells which had transiently down-regulated Foxp3 expression, the possible conversion of conventional CD4 T-cells to so-called induced Treg cells has to be considered. This is excluded, however, because removal of CD25-expressing cells, i.e. all pre-existing Treg cells, abolishes the effect of IL-2, IL-7, or IL-15 mediated up regulation of Foxp3 and CD25. This result cannot be explained by blockade of CD25, which is a component of the IL-2R, because IL-7 and IL-15 use receptors with different light chains.

In the document Zorn, E., et al., IL-2 regulates FOXP3 expression in human CD4+CD25+ regulatory T cells through a STAT-dependent mechanism and induces the expansion of these cells in vivo, Blood 2006.108:1571-1579, it was neither suspected nor suggested that Treg cells transiently down-regulate Foxp3 as a result of cytokine deprivation during recirculation in the blood. Accordingly, it was not envisioned to use cytokine-mediated up-regulation of Foxp3 expression to reveal transiently Foxp3 negative blood-borne Treg cells for diagnostic, analytical or preparative purposes, which would otherwise go undetected, as is presented in the instant invention. Rather, it was suggested that IL-2 treatment could be used in certain disease settings to boost Treg activity, including Foxp3 expression, above the body's steady-state level.

For many routine measurements of lymphocyte populations, including Treg cells, PBMC are not immediately used but the venous blood is kept at room temperature (RT) with an anticoagulant for shipment. Protocols of clinical studies allow for many hours, often including overnight shipment, before analysis is performed. In another setting, often used for experimental purposes, PBMC are prepared from leukocyte reduction chambers as a by-product of platelet concentrate production (Dietz, A. B. et al., A novel source of viable peripheral blood mononuclear cells from leukoreduction system chambers, Transfusion 2006.46: 2083-2089), a process taking several hours, followed by transport to the laboratories at room temperature. Furthermore, PBMC are often cryopreserved for shipment and storage. Importantly, restoration of the Foxp3+ CD25+ (Treg) phenotype by overnight incubation with IL-2 is effective in all of these situations.

It is important to note that "invisible" Treg cells found in peripheral blood can safely be assumed to re-express Foxp3 also in nature when they re-enter tissues and are again supplied with cytokine signals activating the STATS pathway. The current procedure thus visualizes a biologically important cell population, which would otherwise go unnoticed. This is illustrated by the observation that side-by-side comparison of Treg cells present in lymph nodes and blood of the same donor shows not only a much higher frequency in the solid tissue as compared to the blood stream, there is, moreover, no further increase as a result of IL-2 stimulation in the detectable lymph node Treg population, whereas if cultured without IL-2, this frequency declines. In contrast, as mentioned before, the detectable Treg population in the blood sample markedly increases after IL-2 stimulation.

In summary, the concept of the present invention reveals the existence of a large fraction of "invisible" Treg cells in human peripheral blood which by current analytical procedures remain undetected because they do not express sufficient levels of Foxp3 for detection by flow cytometry as a result of cytokine deprivation during their sojourn in the blood. Furthermore, it solves the problem of invisibility of these Treg cells in analytic or preparative procedures by a new method of the invention being named TRICKS (for TReg Identification by CytoKin Stimulation), which allows the identification of these transiently Foxp3 negative Treg cells through stimulation with cytokines. Of note, this method is not only of use for a correct determination of Treg frequencies in PBMC, but can also be used to further characterize these cells, i.e. with regard to the expression of homing receptors such as chemokine receptors and integrins, and for preparative purposes based on high expression of CD25, which is co-regulated with Foxp3, in conjunction with other, established cell surface markers.

Generally CD4 and/or CD25 positive cells may be identified in and/or separated from the sample by general methods known in the art, in particular using monoclonal antibodies specific for CD4 and/or CD25. Separation can be carried out by any cell-sorting technique known in the art, e.g. flow cytometric or magnetic bead based methods.

According to a preferred embodiment of the invention said sample is untreated human blood or is obtained from the human blood by isolation of peripheral blood mononuclear cells (PBMC) from the human blood.

The analytic compound specific for Forkhead-Box-Protein P3 (Foxp3) is preferably a monoclonal antibody comprising a fluorochrome.

Cells, which are CD25 and/or CD4 positive, may be identified and/or isolated before, at the same time, or after step A2), B2), or C2),.

The compound capable of activating STAT5 is preferably selected from the group consisting of IL-2, IL-7 and IL-15, in particular is IL-2.

In a particularly preferred embodiment of the invention, PBMC are isolated from a human peripheral blood sample by density centrifugation, and used either immediately or after frozen storage according to standard methods before being cultured for 1- 48 hours, preferably 16 - 24 hours, in the presence of a cytokine, preferentially a cytokine of the family which activates the STAT5 signaling pathway, preferentially IL-2, 1L-7 or IL-15, more preferentially IL-2 at a dose above 1 U/ml, preferentially above 200 U/ml, before proceeding with phenotypic characterization by standard means, i.e. using monoclonal antibodies (mAb) specific for cell surface markers (usually CD4 and CD25) and for Foxp3 to identify, quantify and further characterize Treg cells. For the preparation of live Treg cells which precludes intracellular staining of Foxp3, cells expression CD4 and high levels of CD25 can be isolated by flow cytometric or other cell-sorting techniques.

In another particularly preferred embodiment, venous blood is supplemented with a cytokine, preferentially a cytokine which activates the STAT5 pathway, preferentially IL-2,IL-7 or IL,-15, more preferentially IL-2, at a dose above 1 U/ml, preferentially above 200 U/mi, before storage and shipment prior to further processing as above.

In the following the invention is explained in more detail by way of non-limiting figures and examples. The figures show:

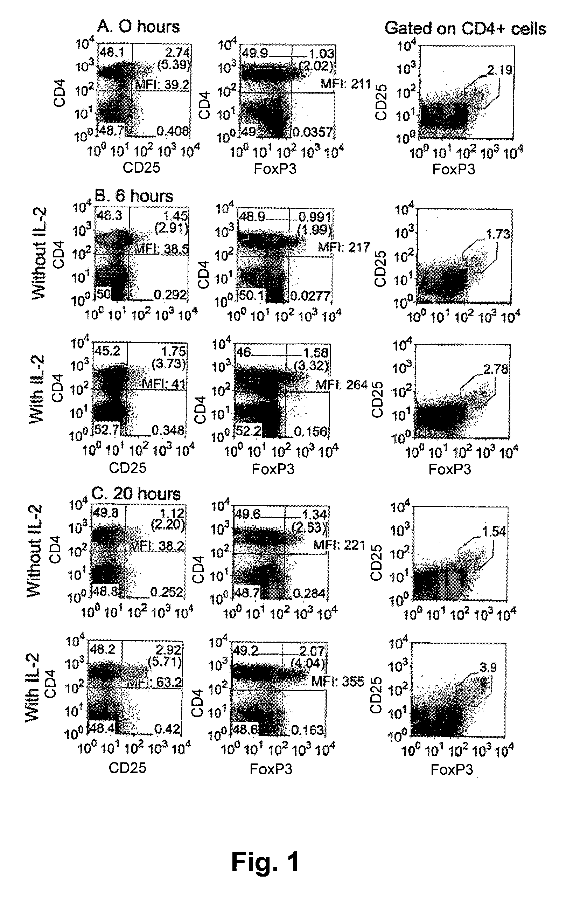

- Fig. 1:: Expression levels of Foxp3 and CD25 and frequencies of Treg after stimulation of freshly prepared PBMC cultures with IL-2. Effect of overnight stimulation of freshly prepared PBMC cultures with IL-2 on the frequencies of Treg cells and the expression levels of the Treg markers Foxp3 and CD25. PBMC from a healthy donor were either analyzed immediately (A) or after 6 (B) or 20 hours (C) with or without 200/ml IL-2, for cell surface expression of CD4 and CD25, and for intracellular expression of Foxp3. Numbers represent percentages in the respective fields. Numbers in brackets represent frequencies of Foxp3 positive cells among CD4 T-cells.

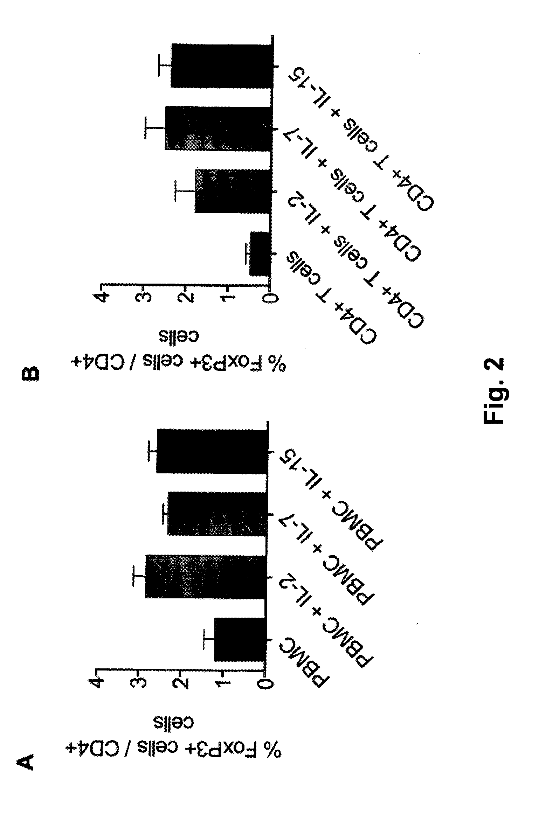

- Fig. 2:: Up regulation of Foxp3 in a subset of CD4 positive T-cells after stimulation with STAT5-activating cytokines. STAT5-activating cytokines induce upregulation of Foxp3 directly in a subset of CD4 T-cells. Unseparated PBMC (A) or purified CD4 T-cells were cultured overnicht with 200 U/ml IL-2, or with 20ng of IL-7 or IL-15.

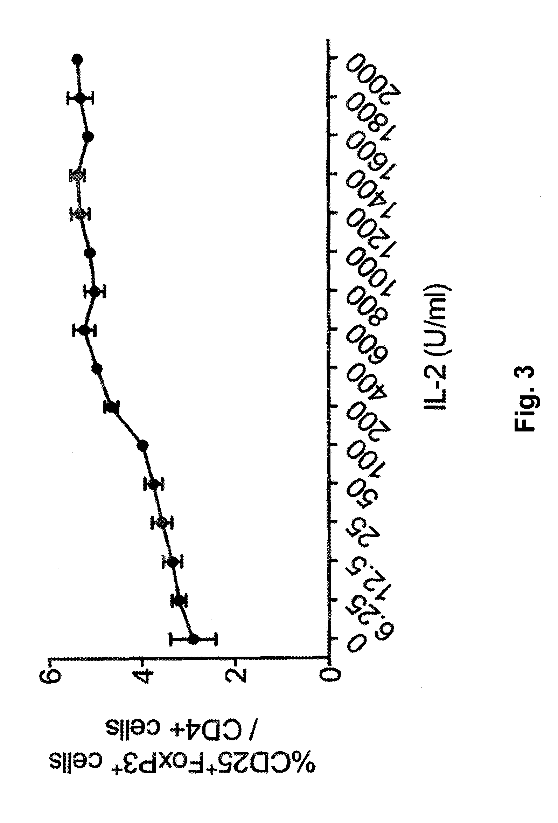

- Fig. 3:: Dose dependent induction of Foxp3. Dose-dependent increase in visible Treg cells. PBMC were cultured overnight in the presence of graded concentrations of IL-2, followed by flow-cytometric analysis of cell surface expression of CD4 and CD25, and for intracellular expression of Foxp3.

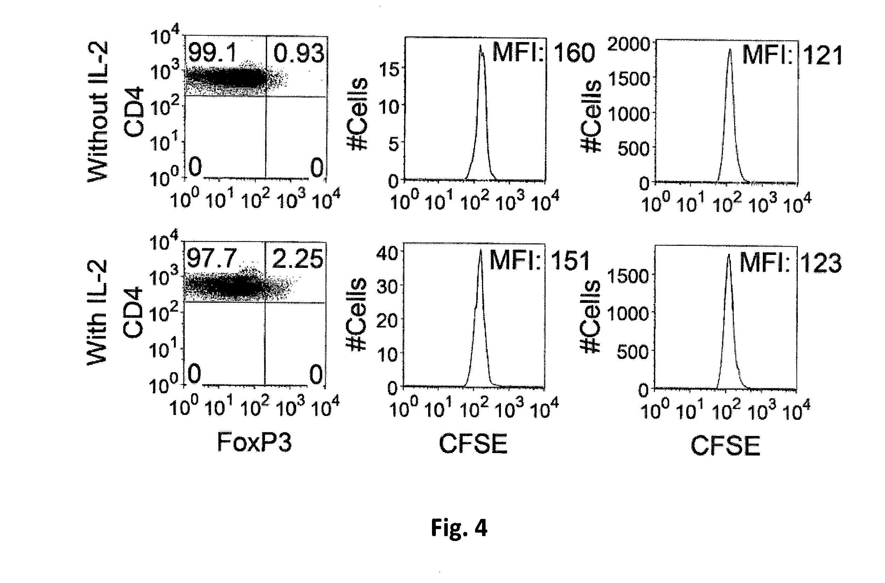

- Fig. 4:: Data showing that the increase in Foxp3 positive cells is not due to cell division. PBMC were labelled with the covalent dye CFSE, and cultured overnight with or without 200 U/ml IL-2. They were stained for CD4 and Foxp3. CFSE fluorensence is shown for Foxp3-negative (left) and Foxp3-positive (right).

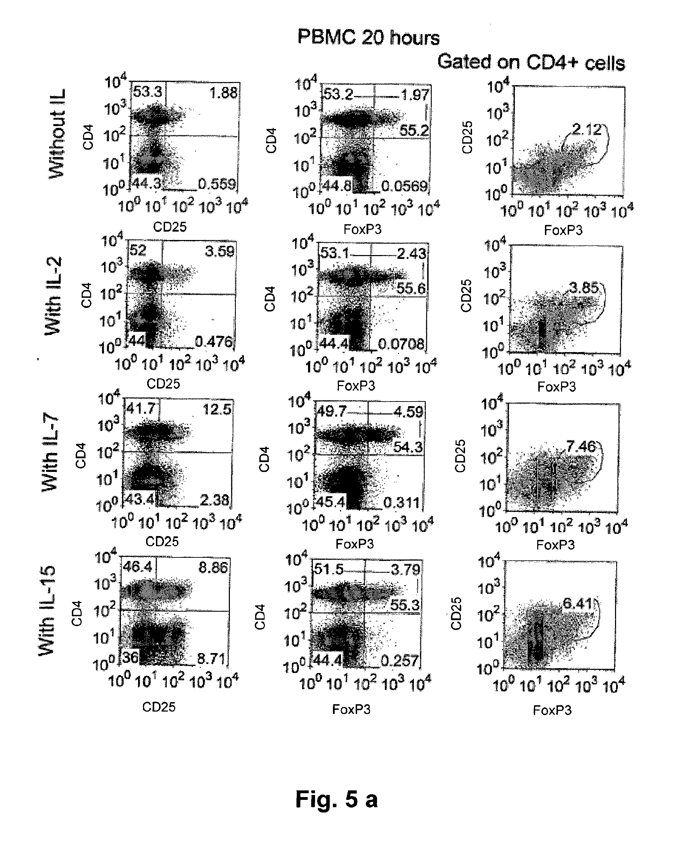

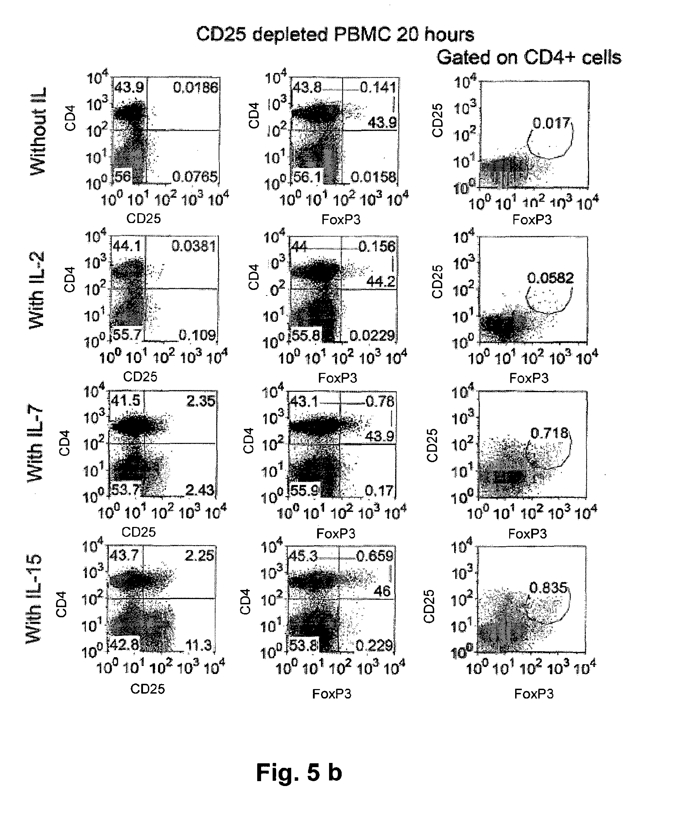

- Fig. 5:: Data showing that the CD4 positive cells responding to IL-2, IL-7, or IL-15 treatment with Foxp3 up regulation express CD25, i.e. are Treg cells. PBMC were depleted of CD25 expressing cells by magnetic separation (or not), and cultured overnight with 200 U/ml IL-2, IL-7 or IL-15 before analysis as in Figure 1. Numbers in brackets are percent Foxp3+ or all CD4+ T-cells.

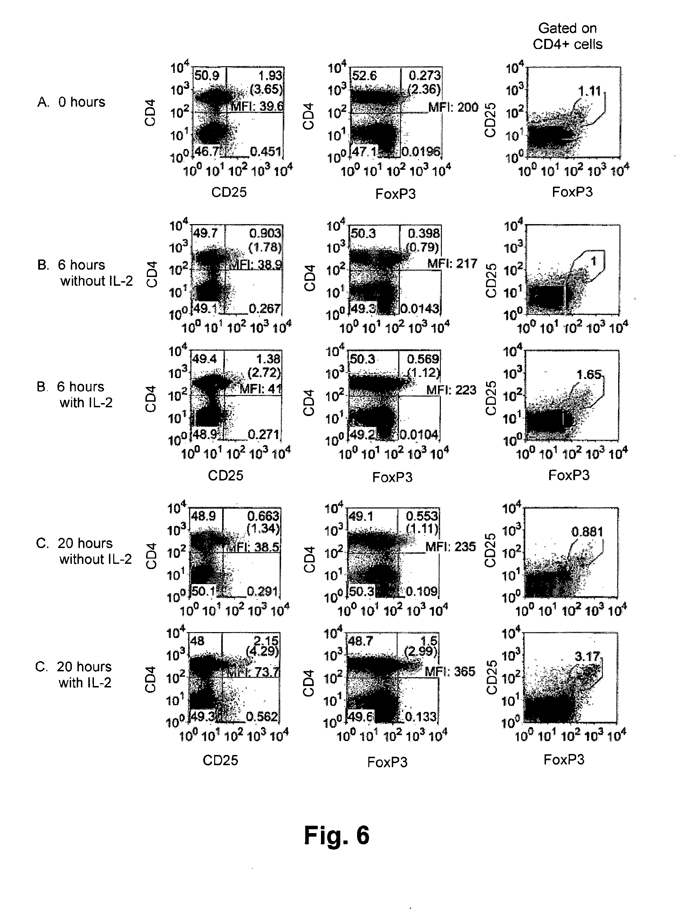

- Fig. 6:: Analysis of previously frozen PBMC. The experiment was performed as in Figure 1, but with previously frozen PBMC.

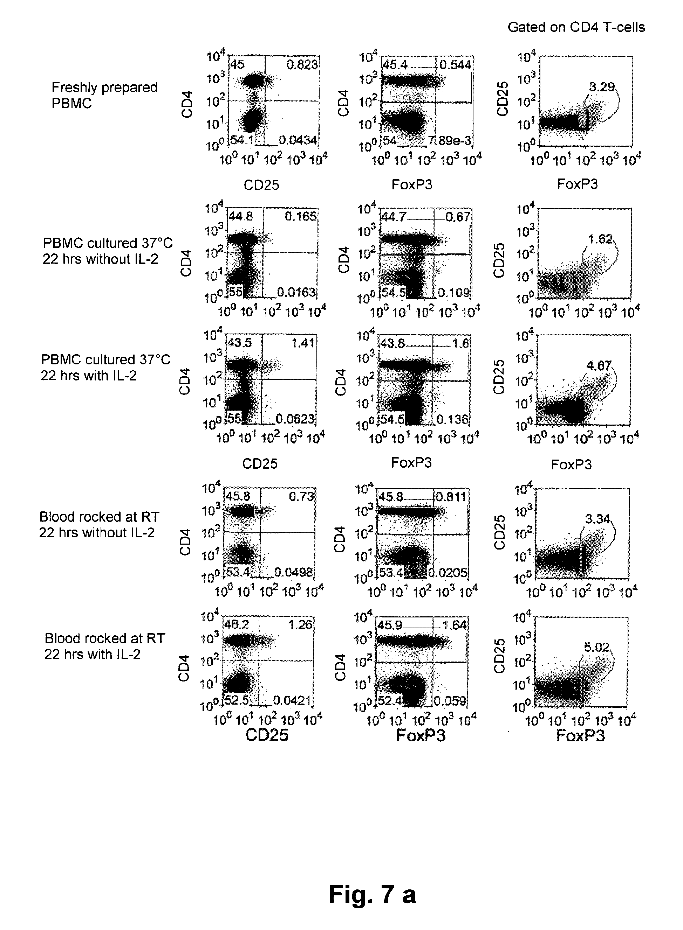

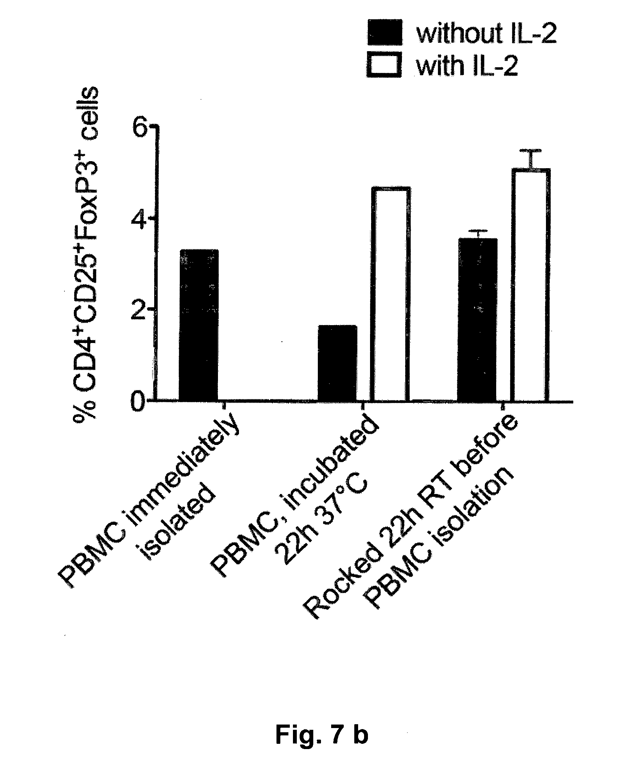

- Fig. 7:: Data showing that the inclusion of IL-2 during transport of heparinized blood at RT (20°C) increases detectable Treg cells. Freshly drawn heparinized venous blood was either used for immediate PBMC preparation, or was gently rocked at RT with or without 400 U/ml of IL-2 for 22 hrs before PBMC preparation. Dot plots show raw data, the bar graph summarizes results. Triplicates were performed for the 22 hour rocked groups, of which means and standard deviations are shown.

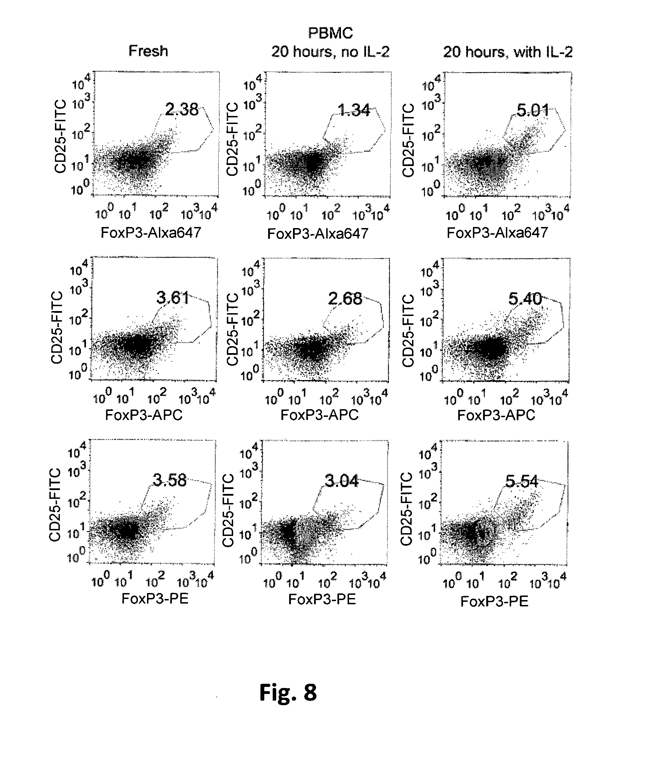

- Fig. 8:: The increase in detectable Treg cells after culture in the presence of IL-2 with three different staining protocols. PBMC were stained as described in Example 1, using Alexa-647-conjugated anti-Foxp3 (clone 259D, Biolegend) (top row), APC-conjugated anti-Foxp3 (clone 236/A/E7, eBioscience) (center row), or PE conjugated anti-Foxp3 (clone 236A/E7, eBioscience) for Foxp3 detection. Dot plots were gated on CD4+ lymphocytes.

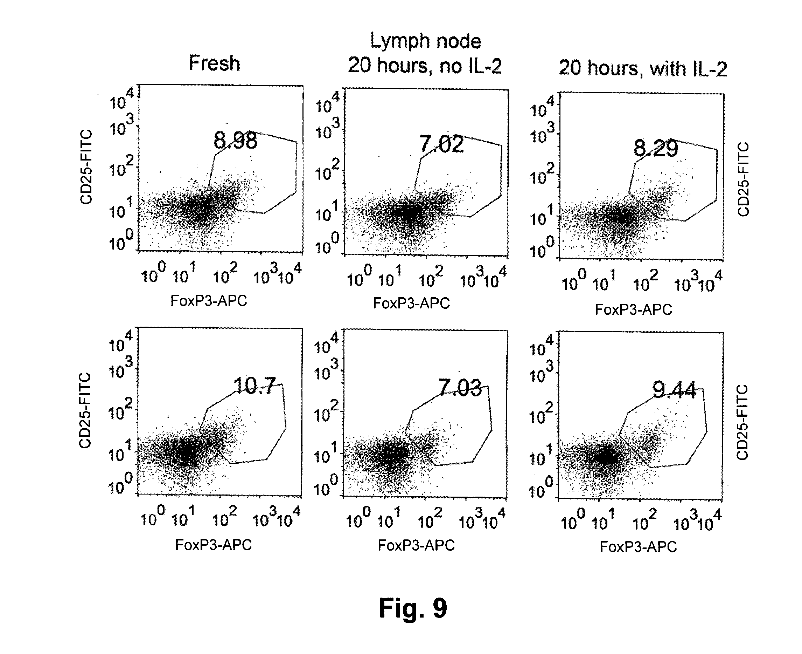

- Fig. 9:: Data showing that no increase in detectable Treg cells after culture in the presence of IL-2 is observed in lymph node cells. Lymph node cells from the same donor whose PBMC were analyzed in Figure 8 were stained as in Fig 8, center and bottom rows. Dot plots were gated on CD4+ lymphocytes.

### Example 1: Comparative example using conventional Treg analysis and the protocol of the invention starting with freshly isolated PBMC

For the determination of Treg frequencies, the present invention as well as conventional state-of-the-art measurements use PBMC isolated from heparinized venous blood by centrifugation over a density gradient (lymphocyte separation medium Pancoll human, PAN-BIOTECH GmbH, Aidenbach, Germany) following the manufacturer's instructions.

PBMCs were cultured in 48-well tissue culture plates (Greiner bio-one, Frickenhausen, Germany), in which 0,2, 0,5, or 1 ml of cells adjusted to 1 Mio/ml were cultured per well in the three types of wells mentioned, using enriched RPMI 1640 culture medium (GIBCO/Invitrogen, Long Island, NY, USA) supplemented with 10% autologous serum or commercially available pooled human AB serum (Sigma-Aldrich), with essentially the same results.

The frequency of Treg cells was determined by 3-colour immunofluorescence and flow cytometry, using fluorochrome-conjugated mAb specific for CD4, CD25 and Foxp3. For the staining procedure, the cells were suspended at 1 x 10⁶/ml in staining buffer (PBS, 0,1 % BSA, 0,2 % NaN₃), and the CD4 and CD25 specific mAb were added to 0,2 ml of this suspension. After 20 min. on ice, cells were washed with staining buffer by centrifugation (4°C, 1600 rpm). For intracellular staining of Foxp3, the cells were then suspended in Fix/Perm (eBioscience), and stained with the appropriate antibody diluted in Perm/Wash (eBioscience).

The following fluorochrome-conjugated mAb were used: CD4 (clone RPA-T4, PECy5,1:300, BioLegend), CD25 (clone M-A251, PE,1:25,-BD), FoxP3 (clone 259D), Alexa647,1:50, BioLegend).

After the final wash, PBMC were resuspended in 0,1 ml staining buffer and analyzed on a FACS Calibur flow cytometer (Becton Dickinson, Mountain View, CA). Data were then analyzed using FlowJo software (TreeStar). They were displayed as dot plots wherein each cell is represented by a dot, and logarithmic fluorescence intensity of two markers (e.g. CD4 and Foxp3) defines the position of each dot. Horizontal and vertical lines divide the dot plot into four quadrants, with lower left containing cells expressing neither marker, upper right both markers, and upper left and lower right only one of the two markers studied. Marker-positive cell populations can also be defined by a window encompassing a "cloud" of dots.

Analysis of PBMC prepared from venous blood from a healthy donor is shown in Figure 1. Staining was performed directly after isolation of PBMC (Fig. 1A), and after six (Fig. 1B) or 20 hours (Fig. 1C) of incubation in the absence or in the presence of 200 U/ml recombinant human 1 L-2 (Novartis). Numbers represent percentages in the respective fields. Numbers in brackets represent frequencies of Foxp3 positive cells among CD4 positive T-cells.

As is readily seen, the frequency of Foxp3 expressing and of Foxp3 and CD25 coexpressing cells within the CD4 T-cell subset, i.e. cells identified as Treg cells, almost doubled after overnight culture in the presence, but further decreased in the absence of IL-2. This positive effect became first apparent alter 6 hours of culture. In addition to frequencies of cell populations, also the mean fluorescence intensities (MFI) observed for Foxp3 and CD25 in the positive populations are shown. These are proportional to the amount of the respective proteins expressed by the marker-positive cells. It is obvious that not only the frequencies, but also the expression levels of Foxp3 and CD25 expression per cell were increased as a result of IL-2 stimulation.

Compiled data from four randomly chosen healthy donors obtained in an identical fashion are summarized in Table 1. It can be seen that not only the frequency of Treg marker-positive cells, but also the level of marker expression were increased as a result of IL-2 treatment. The top group shows data for PBMC isolated directly from venous blood. The bottom group shows data for PBMC isolated from leukocyte reduction chambers obtained as a by-product of platelet concentration production. (A = CD25+/CD4+ cells [%], B = MFI of CD25 in CD4+ CD25+ cells, C = Foxp3+/CD4+ cells [%], D = MFI of Foxp3 in CD4+ Foxp3+ cells, E = Treg (CD25+Foxp3+ cells/CD4+ cells [%])

**Table 1:**

| Donor | Time | A | | B | | C | | D | | E | |
|---|---|---|---|---|---|---|---|---|---|---|---|
| | [h] | no | | no | | no | | no | | no | |
| | | IL-2 | IL-2 | IL-2 | IL-2 | IL-2 | IL-2 | IL-2 | IL-2 | IL-2 | IL-2 |
| D263 | 0 | 4,56 | | 38,2 | | 1,33 | | 211 | | 2,06 | |
| | 2 | 1,77 | 17,0 | 36,2 | 37,2 | 0,92 | 1,05 | 198 | 215 | 1,18 | 1,36 |
| | 6 | 2,52 | 3,11 | 37,5 | 41,4 | 1,35 | 2,51 | 215 | 257 | 1,64 | 2,69 |
| | 20 | 1,93 | 3,82 | 38,2 | 54,2 | 1,34 | 3,31 | 204 | 382 | 1,41 | 3,28 |
| D264 | 0 | 5,39 | | 39,2 | | 2,02 | | 211 | | 2,19 | |
| | 2 | 2,81 | 2,83 | 38,2 | 38,2 | 1,40 | 1,99 | 213 | 219 | 1,28 | 1,70 |
| | 6 | 2,91 | 3,73 | 38,5 | 41,0 | 1,99 | 3,32 | 217 | 264 | 1,73 | 2,78 |
| | 20 | 2,20 | 5,71 | 38,2 | 63,2 | 2,63 | 4,04 | 221 | 355 | 1,54 | 3,90 |
| D265 | 0 | 4,32 | | 39,2 | | 2,14 | | 221 | | 2,60 | |
| | 2 | 2,23 | 2,68 | 37,9 | 38,2 | 2,23 | 2,44 | 217 | 217 | 2,06 | 2,33 |
| | 6 | 2,51 | 3,27 | 39,2 | 42,6 | 2,51 | 3,75 | 229 | 305 | 2,31 | 3,36 |
| | 20 | 2,54 | 5,04 | 40,3 | 59,9 | 2,72 | 4,35 | 239 | 385 | 2,29 | 4,33 |
| D266 | 0 | 8,33 | | 36,8 | | 1,38 | | 213 | | 1,80 | |
| | 2 | 3,25 | 2,47 | 37,5 | 37,2 | 2,24 | 2,33 | 207 | 213 | 1,79 | 1,61 |
| | 6 | 3,50 | 4,62 | 37,9 | 40,7 | 2,56 | 3,48 | 221 | 271 | 1,96 | 2,85 |
| | 20 | 2,86 | 5,36 | 40,7 | 56,2 | 1,73 | 4,21 | 231 | 346 | 1,51 | 3,79 |
| D267 | 20 | 5,46 | 8,08 | 29,5 | 49,1 | 2,31 | 6,70 | 221 | 273 | 2,16 | 5,53 |
| | 46 | 4,61 | 8,02 | 30,3 | 60,9 | 1,08 | 3,83 | 224 | 407 | 1,37 | 4,40 |
| D254 | 0 | 5,87 | | 37,2 | | 0,58 | | 198 | | 3,33 | |
| | 24 | 6,26 | 8,94 | 39,6 | 55,2 | 0,47 | 3,14 | 175 | 172 | 2,52 | 7,72 |
| D256 | 0 | 2,85 | | 67,3 | | 1,08 | | 284 | | 2,21 | |
| | 24 | 0,98 | 3,74 | 63,2 | 87,4 | 1,17 | 3,83 | 274 | 365 | 1,36 | 4,21 |
| D257 | 0 | 4,57 | | 70,4 | | 3,02 | | 237 | | 3,35 | |
| | 24 | 3,64 | 8,01 | 66,1 | 96,5 | 4,09 | 7,23 | 239 | 340 | 3,10 | 6,79 |

### Example 2: Cellular and Cytokine Requirements for the Recovery of Foxp3 Expression

The experiment shown in Fig. 2 asked the question whether the recovery of Foxp3 expression requires additional cell types such as monocytes contained within the PBMC preparation, and whether additional cytokines beyond IL-2, which activate the STAT5 pathway of signal transduction, would be able to promote recovery of Foxp3 expression. Fig. 2A shows results obtained with unseparated PBMC and Fig. 2B results from purified CD4 positive cells. CD4 T-cells were purified by using a CD4 T-cell purification kit (CD4+ T Cell Isolation Kit 11, Miltenyl Biotec) and were then cultured as given in previous experiments for 20 hours in the presence of 200 U/ml IL-2, 20 pg/ml IL-7, or 20 pg/m1 IL-15, before determining the frequency of Foxp3+ cells among CD4 T-cells. It is apparent that Foxp3 negative Treg cells contained within the CD4 T-cell population are able to respond to IL-2 with re-expression of Foxp3. Surprisingly, IL-7 and IL-15 are also highly efficient in providing this effect.

### Example 3: Dose-response relationship

PBMC were cultured with titrated concentrations of IL-2 for 20 hours as in Example 1. Frequencies of Foxp3+CD25+ cells within the CD4 T-cell compartment were determined as in example 1, and are graphically displayed in Fig. 3. It is found that an increase in detectable Treg cells is apparent with as little as 6,25 U/ml of IL-2, followed by a dose-dependent further increase.

### Example 4: Kinetics of the response

To determine the length of time required for an optimal effect, purified CD4 T-cells were incubated with or without 200 U/ml of IL-2 for various lengths of time (Table I). While in the absence of IL-2, the frequency of Foxp3 positive cells declined, it increased in its presence, reaching a plateau after 20 hours. Extending the culture period to 46 hours did not show any further increase over the overnight incubation time, as evident from Table I, above (donor D267).

### Example 5: Increase in Foxp3+ cells after incubation with IL-2 is not due to cell division

The method of carboxyfluorescein succinimidyl ester (CFSE) dye dilution (see e.g. Tabares, P. et al., Human regulatory T cells are selectively activated by low-dose application of the CD28 superagonist TGN1412/TAB08, Eur J Immunol 2014.44: 1225-:1236) was employed to determine the proliferation history of Foxp3+ cells recovered from IL-2 stimulated overnight cultures. In this method, which is well known to skilled immunologists, individual cell divisions are easily visualized by flow cytometry as a 50% reduction of fluorescence intensity when the label is distributed to the two daughter cells. In this experiment, which is shown in Fig.4, the frequency of Foxp3+ cells among CD4 T-cells was 2,4 fold higher in 20 hour cultures containing 200 U/ml IL-2 as compared to cultures without IL-2. Nevertheless, the cells display a single peak of CFSE Label of the same MFI under both conditions, indicating that no cell divisions had taken place during the culture period.

Thus, this experiment reveals no cell division at all in the increased population of Treg cells identifiable in IL-2 treated cultures, thereby establishing that the effect of IL-2, i.e. doubling in the frequency of Foxp3+ cells, is by up-regulation of Foxp3 expression in previously "invisible" Treg cells rather than by stimulation of Treg cell proliferation.

For the results in Fig. 4 the PBMC were stained for CD4 and Foxp3. The left hand CFSE diagrams are for Foxp3 negative and the right hand diagrams for Foxp3 positive CD4 T-cells.

### Example 6: Increase in Foxp3+ cells after incubation with IL-2, IL-7, or IL-15 is not due to conversion of conventional CD4 T-cells

To remove Treg cells which had transiently down-regulated Foxp3 from PBMC, cells expressing CD25 (which is expressed by all Treg cells and a few activated conventional CD4 T-cells) were removed by mAb-mediated magnetic depletion. As seen in Figure 5, this eliminated the capacity of IL-2 to increase the frequency of Foxp3+ cells, indicating that culture in the presence of IL-2, and, to a large extent, of IL-7 or IL-15 to increase the frequency of Foxp3+ CD25+ cells, indicating that culture in the presence of these STAT5-activating cytokines allows re-expression of Foxp3 in Treg cells which had transiently lost Foxp3 expression but still express sufficient CD25 to allow depletion with CD25-specific mAb. Culturing was carried out overnight with 200 U/ml IL-2, IL-7 or IL-15 before analysis as in Fig. 1.

### Example 7: Culture with IL-2 increases detectable Treg cells in frozen / thawed PBMC

PBMC were stored frozen in DMSO containing medium at -80°C, and thawed prior to analysis. This procedure is routinely applied by researchers working with PBMC, which are aliquoted and frozen for later analysis. As can be seen in Fig. 6, the detectable Treg frequency in these cells was also considerably enhanced by culture with IL-2. The experiments were performed as in Figure 1, but with previously frozen PBMC.

### Example 8: Inclusion of IL-2 during transport up-regulates CD25 and Foxp3 expression

Freshly drawn heparinized venous blood was either used for immediate PBMC preparation, or transport at 20°C was mimicked by gently rocking the heparinized blood with or without 400 U/ml of IL-2 for 22 h before PBMC preparation. As can be seen in Figure 7, this procedure also increased the frequency of detectable Treg cells. The dot plots show raw data and the bar graph summarized results.

### Example 9: The increase in detectable Treg frequency is independent of the staining protocol employed.

PBMC were stained without pre-culture, or after 20 h culture in the presence or absence of 200 U/ml of IL-2. In addition to the Alexa647 conjugate of the anti-Foxp3 mAb clone 259B (Biolegend, top row in Fig. 8) used so far, two different antibody-fluorochrome conjugates of an alternative clone were used for the detection of Foxp3: Clone 236A/E7 conjugated with phycoerythrin (PE, bottom row in Fig. 8) or with allophycocyanin (APC, both from eBioscience, center row in Fig. 8). Fig. 8 shows that while the latter two mAb yielded superior results in the detection of Foxp3, the phenomenon that culture with IL-2 increases the frequency of detectable Treg among CD4 T-cells, whereas culture without IL-2 leads to a further decrease was clearly visible with all three staining procedures. The dot plots were gated on CD4+ lymphocytes.

### Example 10: Treg cells in blood, but not in lymph nodes are "invisible" because of cytokine deprivation.

Lymph nodes were collected from the para-iliac region of renal transplant recipients at the Academic Medical Center, Amsterdam, The Netherlands. Paired peripheral blood samples were collected before the transplantation procedure. Cells were frozen in IMDM supplemented with 10% DMSO, 20% FCS, penicillin, streptomycin, and 0,00036% mercaptoethanol. The study was approved by the local ethics committee at the Academic Center at the University of Amsterdam.

Cells shown in Figure 9 were stained without pre-culture, or after 20 h culture in the presence or the absence of 200 U/ml of IL-2, using the anti-Foxp3 mAb clone 236A/E7 conjugated with phycoerythrin (EP) or with allophycocyanin (APC, both from eBioscience). The lymph node cells were from the same individual as the PBMC in Fig. 8. It is noted that in contrast to these, culture with IL-2 did not lead to a further increase in Treg frequency, whereas as predicted, culture without IL-2 led to their apparent reduction.

**Table I. Compiled data of samples analysed as in Figure 1. Top group: PBMC isolated directly from venous blood. Bottom group: PBMC isolated from leukocyte reduction chambers obtained as a by-product of platelet concentrate production.**

| **Donor** | **Time (hours)** | **% CD25** + **cells / CD4+ cells** | | **MFI CD4+ CD25+ cells** | | **% FoxP3**+ **cells** / **CD4**+ **cells** | | **MFI CD4+ FoxP3**+ **cells** | | **% Tregs** | |
|---|---|---|---|---|---|---|---|---|---|---|---|
| | | No IL-2 | IL-2 | No IL-2 | IL-2 | No IL-2 | IL-2 | No IL-2 | IL-2 | No IL-2 | IL-2 |
| **D263** | 0 | 4.56 | - | 38.2 | - | 1.33 | - | 211 | - | 2.06 | - |
| | 2 | 1.77 | 17.01 | 36.2 | 37.2 | 0.92 | 1.05 | 198 | 215 | 1.18 | 1.36 |
| | 6 | 2.52 | 3.11 | 37.5 | 41.4 | 1.35 | 2.51 | 215 | 257 | 1.64 | 2.69 |
| | 20 | 1.93 | 3.82 | 38.2 | 54.2 | 1.34 | 3.31 | 204 | 382 | 1.41 | 3.28 |
| **D264** | 0 | 5.39 | - | 39.20 | - | 2.02 | - | 211 | - | 2.19 | - |
| | 2 | 2.81 | 2.83 | 38.20 | 38.20 | 1.40 | 1.99 | 213 | 219 | 1.28 | 1.70 |
| | 6 | 2.91 | 3.73 | 38.50 | 41.00 | 1.99 | 3.32 | 217 | 264 | 1.73 | 2.78 |
| | 20 | 2.20 | 5.71 | 38.20 | 63.20 | 2.63 | 4.04 | 221 | 355 | 1.54 | 3.90 |
| **D265** | 0 | 4.32 | - | 39.20 | - | 2.14 | - | 221 | - | 2.60 | - |
| | 2 | 2.23 | 2.68 | 37.90 | 38.20 | 2.23 | 2.44 | 217 | 217 | 2.06 | 2.33 |
| | 6 | 2.51 | 3.27 | 39.20 | 42.60 | 2.51 | 3.75 | 229 | 305 | 2.31 | 3.36 |
| | 20 | 2.54 | 5.04 | 40.30 | 59.90 | 2.72 | 4.35 | 239 | 385 | 2.29 | 4.33 |
| **D266** | 0 | 8.33 | - | 36.80 | - | 1.38 | - | 213 | - | 1.80 | - |
| | 2 | 3.25 | 2.47 | 37.50 | 37.20 | 2.24 | 2.33 | 207 | 213 | 1.79 | 1.61 |
| | 6 | 3.50 | 4.62 | 37.90 | 40.70 | 2.56 | 3.48 | 221 | 271 | 1.96 | 2.85 |
| | 20 | 2.86 | 5.36 | 40.70 | 56.20 | 1.73 | 4.21 | 231 | 346 | 1.51 | 3.79 |
| **D267** | 20 | 5.46 | 8.08 | 29.50 | 49.13 | 2.31 | 6.70 | 221 | 273 | 2.16 | 5.53 |
| | 46 | 4.61 | 8.02 | 30.33 | 60.87 | 1.08 | 3.83 | 224 | 407 | 1.37 | 4.40 |
| | | | | | | | | | | | |

| **Donor** | **Time (hours)** | **% CD25 + cells** / **CD4+ cells** | | **MFI CD4+ CD25+ cells** | | **% FoxP3**+ **cells** / **CD4+ cells** | | **MFI CD4+ FoxP3**+ **cells** | | **% Tregs** | |
|---|---|---|---|---|---|---|---|---|---|---|---|
| | | No IL-2 | IL-2 | No IL-2 | IL-2 | No IL-2 | IL-2 | No IL-2 | IL- 2 | No IL-2 | IL-2 |
| **D254** | 0 | 5.87 | - | 37.20 | - | 0.58 | - | 198 | - | 3.3 | - |
| | 24 | 6.26 | 8.94 | 39.60 | 55.20 | 0.47 | 3.14 | 175 | 172 | 2.52 | 7.72 |
| **D256** | 0 | 2.85 | - | 67.30 | - | 1.08 | - | 284 | - | 2.21 | - |
| | 24 | 0.98 | 3.74 | 63.20 | 87.40 | 1.17 | 3.83 | 274 | 365 | 1.36 | 4.21 |
| **D257** | 0 | 4.57 | - | 70.40 | - | 3.02 | - | 237 | - | 3.35 | - |
| | 24 | 3.64 | 8.01 | 66.10 | 96.50 | 4.09 | 7.23 | 239 | 340 | 3.10 | 6.79 |

## Claims

1. Method for the in-vitro determination of regulatory T-cells (Treg cells) in a sample obtained from human blood with the following steps:
A1) said sample is supplemented with a compound capable of activating the Signal Transducer and Activator of Transcription 5 (STAT5) and in an amount capable for such activation, and cultured for a predetermined cultivation duration under conditions which keep T-cells comprised in said sample viable, wherein the cultivation is carried out either during said supplementing or after said supplementing,
A2) after cultivation of said sample an analytical compound specific for CD25 and/or Forkhead-Box-Protein P3 (Foxp3) is added to said sample and the cell frequency of CD25 and/or Foxp3 positive cells is determined in said sample, in particular by determining the ratio of the amount of CD25 and/or Foxp3 positive cells to the amount of CD4 positive cells.

2. Method for preparing a composition, in particular a pharmaceutical composition, comprising a predetermined amount of viable Treg cells with the following steps:
B1) a sample is obtained from human blood and subjected to an in-vitro cultivation under conditions which keep T-cells comprised in said sample viable, said cultivation being performed during or after supplementing said sample with a compound capable of activating the Signal Transducer and Activator of Transcription 5 (STAT5),
B2) then an analytic compound specific for CD25 is added and the frequency of CD25 positive cells is determined, in particular by determining the ratio of the amount of CD25 positive cells to the amount of CD4 positive cells.
B3) then a fraction of said sample is taken, wherein the fraction is calculated from the frequency of CD25 positive cells such that the fraction comprises the predetermined amount of viable Treg cells, and said fraction is optionally prepared for administration.

3. Method for preparing a composition, in particular a pharmaceutical composition, comprising a predetermined amount of viable Treg cells with the following steps:
C1) a sample is obtained from human blood and subjected to an in-vitro cultivation under conditions which keep T-cells comprised in said sample viable, said cultivation being performed during or after supplementing said sample with a compound capable of activating the Signal Transducer and Activator of Transcription 5 (STAT5),
C2) then an analytic compound specific for Foxp3 is added to a first fraction of said sample and the frequency of Foxp3 positive cells is determined, in particular by determining the ratio of the amount of Foxp3 positive cells to the amount of CD4 positive cells.
C3) then a second fraction of said sample is taken, wherein the amount of the second fraction is calculated from the frequency of Foxp3 positive cells in the first fraction such that the second fraction comprises the predetermined amount of viable Treg cells, and said second fraction is optionally prepared for administration.

4. Method according to one of the claims 1 to 3, wherein said sample is untreated human blood or obtained from the human blood by isolation of peripheral blood mononuclear cells (PBMC) from the human blood.

5. Method according to one of the claims 1 to 4, wherein the analytic compound specific for Forkhead-Box-Protein P3 (Foxp3) is a monoclonal antibody comprising a marker, preferably a fluorochrome.

6. Method according to one of the claims 1 to 5, wherein additionally cells, which are CD25 and/or CD4 positive, are identified and/or isolated before, at the same time, or after step A2), B2), or C2),.

7. Method according to one of the claims 1 to 6, wherein the compound capable of activating STAT5 is selected from the group consisting of IL-2, IL-7 and IL-15, in particular is IL-2.

8. Method according to one of the claims 1 to 7, wherein CD25 positive, and optionally CD4 positive, Treg are separated from said sample.

9. Method according to one of the claims 1 to 8, wherein the cultivation is carried out for at least 1 h, preferably at least 6 h, more preferably at least 16 h, and up to 48 hours or longer, most preferably for 16 to 24 hours.

10. Method according to one of the claims 1 to 9, wherein the compound capable of activating STAT5 is added at a dose of at least 1 U/ml, preferably at least 5 U/ml, more preferably at least 10 U/ml, even more preferably at least 50 U/ml, most preferably at least 200 U/ml.

11. Composition obtained with a method according to one of the claims 2 to 10.

12. Composition according to claim 11 for the treatment of a condition induced by too low levels of Treg in an organism.
